# EUROPÄISCHE PATENTSCHRIFT

(11) **EP 3 402 447 B1**
(45) Veröffentlichungstag und Bekanntmachung des Hinweises auf die Patenterteilung: **30.08.2023**
(21) Anmeldenummer: 17700361.3
(22) Anmeldetag: 13.01.2017
(51) Int. Cl.: A61F 5/28, A61F 5/30, A61F 13/10

(54) **EPICONDYLITIS-PELOTTE**
EPICONDYLITIS PRESSURE PAD
PELOTTE D'ÉPICONDYLITE

(30) Priorität: 13.01.2016 DE 102016000490
(43) Veröffentlichungstag der Anmeldung: 21.11.2018
(73) Patentinhaber: Bauerfeind AG, 07937 Zeulenroda-Triebes (DE)
(72) Erfinder: BAUERFEIND, Hans B., 07937 Zeulenroda-Triebes (DE); SCHEUERMANN, Rainer, 24223 Schwentinental (DE); BÖCKELMANN, Joachim, 47906 Kempen (DE)
(74) Vertreter: Wohlfahrt, Jan Günther
(86) Internationale Anmeldenummer: PCT/EP2017/050654
(87) Internationale Veröffentlichungsnummer: WO 2017/121844

(56) Entgegenhaltungen:
- EP-A1- 0 485 943
- DE-A1-102008 055 867
- DE-C1- 19 716 705
- DE-T2- 60 224 057
- DE-U1-202014 005 972
- US-A1- 2008 262 536
- US-B1- 6 398 749

## Beschreibung

Die Erfindung bezieht sich auf eine Pelotte für eine Epicondylitis-Spange, Epicondylitis-Bandage oder Epicondylitis-Orthese sowie auf eine Epicondylitis-Spange, eine Epicondylitis-Bandage oder eine Epicondylitis-Orthese umfassend eine erfindungsgemäße Pelotte. Die erfindungsgemäße Pelotte, auch als Epicondylitis-Pelotte bezeichnet, zeichnet sich durch eine Positionierung von fünf Druckpolstern auf der an der Haut anliegenden Oberfläche der Pelotte aus. Die spezifische Positionierung der Druckpolster erlaubt in vorteilhafter Weise die Verwendung der Pelotte sowohl am rechten Arm als auch am linken Arm, ohne dass die Positionierung der Pelotte an der Epicondylitis-Spange, der Epicondylitis-Bandage oder der Epicondylitis-Orthese grundlegend geändert werden muss. Darüber hinaus erlaubt die Positionierung der Druckpolster eine Behandlung sowohl des Tennisellenbogens als auch des Golferellenbogens. Daher betrifft die vorliegende Erfindung auch die Verwendung der erfindungsgemäßen Pelotte zur Behandlung des Tennisellenbogens und/oder des Golferellenbogens.

Eine Epicondylitis ist ein erworbener, schmerzhafter Reizzustand der Sehnenansätze von Muskeln des Unterarms, die an den beiden Knochenvorsprüngen oberhalb des Epikondylus am distalen Teil des Oberarmknochens entspringen. Eine Epicondylitis entsteht durch Überbeanspruchung der Unterarmmuskulatur. Es sind zwei Formen der Epicondylitis bekannt, nämlich erstens die Epicondylitis radialis humeri, auch Tennisellenbogen oder Tennisarm genannt, am äußeren Epicondylus des Oberarmknochens, also dem Strecker des Handgelenks und der Finger und zweitens die Epicondylitis ulnaris humeri, auch Golferellenbogen oder Golferarm genannt, am inneren Epicondylus des Oberarmknochens, also dem Beuger des Handgelenks und der Finger.

Tennisellenbogen und Golferellenbogen werden unter anderem mit entsprechenden Bandagen und Spangen behandelt, wie sie in ihrer Grundform beispielsweise aus den Gebrauchsmustern DE 83 123 60 U1 und DE 94 171 91 U1 bekannt sind. Die DE 197 16 705 C1 und die DE 10 2008 055 867 A1 beschreiben Epicondylitis-Spangen, bei denen spezifisch geformte Pelotten durch Drehen beziehungsweise durch Positionsänderung der Pelotten an die Verwendung entweder am rechten oder am linken Arm angepasst werden. Bei diesen Pelotten ist es nur möglich durch Umpositionieren, insbesondere Drehen der Pelotte an der Epicondylitis-Spange eine indikationsgerechte Versorgung des Patienten sicherzustellen. Dies wird jedoch oftmals falsch oder gar nicht durchgeführt. Während ein Behandlung des Golferellenbogens in der DE 197 16 705 C1 gar nicht vorgesehen ist, ist die Behandlung des Golferellenbogens mit einer Spange aus der DE 10 2008 055 867 A1 nur durch unterschiedliche Anbringungsmöglichkeiten der Pelotte an der Spange möglich.

Die US 2008/262536, die als nächstliegender Stand der Technik angesehen wird, offenbart eine Pelotte mit einer Vielzahl von in Reihen auf der Pelottenoberfläche positionierten Erhebungen.

Der Erfindung liegt die Aufgabe zugrunde, eine Epicondylitis-Pelotte zu schaffen, mit der sich eine einstellbare Druckeinleitung auf den Epicondylus in therapeutisch besonders günstiger Weise unabhängig davon erzielen lässt, ob die Pelotte am rechten oder linken Arm angelegt wird und wobei die Pelotte beim Wechsel des Arms nicht aufwendig ummontiert werden muss. Gleichzeitig liegt der Erfindung die Aufgabe zugrunde, eine Pelotte bereitzustellen, mit der sowohl Tennisellenbogen als auch Golferellenbogen, jeweils an der therapeutisch richtigen Stelle behandelt werden können, insbesondere gleichzeitig behandelt werden können, und zwar wahlweise am rechten oder linken Arm.

Die Erfindung löst das ihr zugrunde liegende technische Problem durch eine Pelotte mit einen Grundkörper, wobei der Grundkörper eine erste Fläche und eine der ersten Fläche gegenüberliegende zweite Fläche aufweist, wobei die erste Fläche des Grundkörpers fünf Druckpolster aufweist und wobei das erste Druckpolster im Mittelpunkt eines Rechtecks liegt, das durch das zweite Druckpolster, das dritte Druckpolster, das vierte Druckpolster und das fünfte Druckpolster gebildet wird gemäß der Ansprüche 1 und 2. Erfindungsgemäß sind die fünf Druckpolster auf dem Grundkörper also symmetrisch positioniert.

Die Druckpolster sind so positioniert, dass sie bei Anwendung der Pelotte auf spezifische Nervenenden drücken und diese massieren, sodass der Schmerz nach Überanstrengung, beispielsweise durch Tennis- oder Golfspielen gelindert wird. Triggerpunkte sind lokal begrenzte Muskelverhärtungen in der Skelettmuskulatur, die lokal druckempfindlich sind und von denen übertragene Schmerzen ausgehen können. Durch die erfindungsgemäße Anordnung der fünf Druckpolster werden Triggerpunkte von Muskeln, die an den Knochenvorsprüngen außen (Epicondylus humeri radii) und innen (Epicondylus humeri ulnae) ansetzen oder wie beim Musculus brachioradialis vorbeiführende Muskeln erreicht. Insbesondere können nicht nur die außen am Ellenbogen befindlichen Triggerpunkte, beispielsweise am Musculus extensor carpi radialis longus und Musculus extensor carpi radialis brevis und am Musculus brachioradialis erreicht werden, die bei einem Tennisellenbogen eine Rolle spielen, sondern auch innenliegende Triggerpunkte, beispielsweise die des Musculus pronator teres, des Musculus palmaris longus, des Musculus flexor carpi ulnaris und des Musculus flexor carpi radialis, die beim Golferellenbogen eine Rolle spielen. Während die beiden äußeren Triggerpunkte also zur Behandlung der Schmerzen eines Tennisellenbogens genutzt werden können, sind die inneren Triggerpunkte zur Behandlung des Golferellenbogens geeignet. Durch die erfindungsgemäße Pelotte können daher sowohl der Tennisellenbogen als auch der Golferellenbogen behandelt werden, auch gleichzeitig behandelt werden, ohne dass die Pelotte in ihrer Position verändert, beispielsweise gedreht werden muss. Zusätzlich ergibt sich durch die erfindungsgemäße Positionierung der fünf Druckpolster, dass die Pelotte ohne Neupositionierung, beispielsweise Drehung der Pelotte am Haltemittel, beispielsweise einer Epicondylitis-Spange oder Epicondylitis-Bandage am rechten und am linken Arm verwendet werden kann. Somit wird in vorteilhafter Weise eine Pelotte bereitgestellt, die schon bei Auslieferung mit dem Haltemittel so verbunden sein kann, dass sie indikationsgerecht und korrekt für jedermann positioniert ist, egal, ob die Pelotte zur Behandlung eines Tennisellenbogens und/oder eines Golferellenbogens, und egal, ob die Pelotte zur Behandlung eines linken Arms oder eines rechten Arms vorgesehen ist.

Im Vergleich zu der Pelotte aus der DE 197 16 705 C1, die drei Erhebungen aufweist, hat die erfindungsgemäße Pelotte nicht nur fünf Druckpolster, sondern die Druckpolster sind auch anders positioniert und beabstandet.

In einer ersten erfindungsgemäßen Ausführungsform weist die Pelotte exakt fünf Druckpolster auf. In einer bevorzugten Ausführungsform besteht die Pelotte aus fünf Druckpolstern.

Die erste Fläche des Grundkörpers weist die Druckpolster auf, und ist somit die Fläche, die beim Anlegen der Pelotte an den Arm dem Arm zugewandt ist. Die gegenüberliegende zweite Fläche ist somit dem Arm abgewandt und kann beispielsweise mit einem Pelottenträger, also einem Trägerelement, das die Pelotte trägt, einer Epicondylitis-Spange, einer Epicondylitis-Bandage oder einer Epicondylitis-Orthese verbunden sein, beispielsweise reversibel, insbesondere über einen Klettverschluss verbunden sein. Bevorzugt weist die zweite Fläche einen Klettflächenabschnitt oder einen Flauschflächenabschnitt auf. Bevorzugt ist die Pelotte von einer weichen, textilen Umhüllung umgeben. Bevorzugt weist die Umhüllung einen Klettflächenabschnitt oder einen Flauschflächenabschnitt auf.

In einer bevorzugten Ausführungsform befinden sich das zweite Druckpolster, das dritte Druckpolster, das vierte Druckpolster und das fünfte Druckpolster jeweils an einer Ecke der ersten Fläche des Grundkörpers.

In einer bevorzugten Ausführungsform wird der Grundkörper aus den fünf Druckpolstern gebildet, die also dabei direkt miteinander verbunden sind, wobei die Verbindungsstellen Vertiefungen zwischen den Druckpolstern ausbilden.

In einer zweiten erfindungsgemäßen Ausführungsform weist der Grundkörper die Grundform in etwa eines Rechtecks auf, insbesondere des Rechtecks, dass durch die vier äußeren Druckpolster gebildet wird, wobei das Rechteck abgerundete Ecken aufweist und wobei die vier abgerundeten Ecken des Grundkörpers durch das zweite Druckpolster, das dritte Druckpolster, das vierte Druckpolster und das fünfte Druckpolster gebildet werden.

Der Grundkörper selbst muss jedoch nicht rechteckig sein, sondern kann eine andere Form haben. Beispielsweise können zwischen den die Eckpunkte bildenden Druckpolstern Einbuchtungen im Grundkörper vorgesehen sein, sodass eine knochenähnliche Grundform des Grundkörpers entsteht.

In einer bevorzugten Ausführungsform weist der Grundkörper zwischen dem zweiten Druckpolster und dem dritten Druckpolster, zwischen dem dritten Druckpolster und dem vierten Druckpolster, zwischen dem vierten Druckpolster und dem fünften Druckpolster und zwischen dem fünften Druckpolster und dem zweiten Druckpolster jeweils eine Einbuchtung auf.

In einer bevorzugten Ausführungsform weist der Grundkörper zwischen den Druckpolstern Vertiefungen auf.

Die Vertiefungen stellen die Übergänge zwischen den nebeneinander liegenden Druckpolstern dar beziehungsweise die Vertiefungen stellen die Grunddicke des Pelottengrundkörpers dar, aus der die Polster hervorragen.

In einer bevorzugten Ausführungsform weist das Rechteck zwei Längsseiten und zwei Breitseiten auf, wobei die Längsseiten mindestens 1,2 mal bis höchstens 2,5 mal so lang sind wie die Breitseiten.

Bevorzugt sind die Längsseiten etwa 1,5 mal bis 2 mal so lang wie die Breitseiten. Bevorzugt sind die Längsseiten als Außenkanten des Grundkörpers etwa 4 bis 8 cm lang, besonders bevorzugt etwa 6cm lang und die Breitseiten als Außenkanten des Grundkörpers etwa 2 bis 5 cm lang, insbesondere etwa 4 cm lang. Bevorzugt sind die Längsseiten als Verbindung von den höchsten Punkten von zwei Druckpolstern etwa 3 bis 5 cm lang, besonders bevorzugt etwa 4 cm lang und die Breitseiten als Verbindung von den höchsten Punkten von zwei Druckpolstern etwa 1 bis 3 cm lang, insbesondere etwa 2 cm lang. Bevorzugt sind die höchsten Punkte eines außen liegenden Druckpolsters und des in der Mitte liegenden Druckpolsters etwa 2 cm voneinander entfernt, insbesondere mindestens 1,8 cm und höchstens 2,3 cm voneinander entfernt.

Bevorzugt ragen die Druckpolster etwa 0,2 cm bis etwa 1 cm, insbesondere etwa 0,5 cm aus dem Grundkörper heraus. Die Pelotte hat bevorzugt an den Druckpolstern eine größte Dicke von etwa 0,5 cm bis etwa 2 cm, insbesondere von höchstens etwa 1 cm.

In einer bevorzugten Ausführungsform weisen die fünf Druckpolster im Längsschnitt eine runde oder ellipsoide Schnittfläche auf, wobei die fünf Druckpolster aus dem Grundkörper blasenartig herausragen.

Die Druckpolster ragen also bevorzugt als abgerundete Ausbuchtungen, die kreisrund oder ellipsoid sein können, aus dem Grundkörper heraus.

Für die Druckausübung reicht es in vorteilhafter Weise vollkommen, dass die Druckpolster eine glatte Oberfläche aufweisen. Die Druckausübung der Druckpolster lässt sich jedoch dadurch weiterhin intensivieren, dass man die Druckpolster mit einer profilierten Oberfläche versieht, was zu einer zusätzlichen Friktion oder Massage führt.

In einer bevorzugten Ausführungsform ist die Pelotte aus einem viscoelastischen Material gefertigt. Geeignete Materialien sind dem Fachmann bekannt.

Die erfindungsgemäße Pelotte kann vor allem beim Sport verwendet werden, beispielsweise beim Tennisspielen oder beim Golfspielen.

Die erfindungsgemäße Pelotte kann, beispielsweise beim Sport, präventiv zur Vermeidung von Beschwerden oder zur Linderung von Beschwerden im Ellenbogenbereich verwendet werden.

Bevorzugt wird die Pelotte nichtmedizinisch verwendet. Es ist aber auch eine medizinisch indizierte Verwendung möglich.

Die vorliegende Erfindung betrifft auch eine Epicondylitis-Spange, Epicondylitis-Bandage oder Epicondylitis-Orthese umfassend eine erfindungsgemäße Pelotte und einen Pelottenträger.

Geeignete Epicondylitis-Spangen, Epicondylitis-Bandagen und Epicondylitis-Orthesen, an die über einen Pelottenträger eine Pelotte und insbesondere die erfindungsgemäße Pelotte befestigt werden kann, sind dem Fachmann hinlänglich bekannt, beispielsweise aus der DE 197 16 705 C1. So kann bei der in der DE 197 16 705 C1 gezeigten Epicondylitis-Spange die dort verwendete dreiecksähnliche Pelotte durch die erfindungsgemäße Pelotte ersetzt werden.

Bei einer solchen Epicondylitis-Spange oder Epicondylitis-Bandage bildet das Spangenelement oder das Bandagenelement den Pelottenträger, der die Pelotte hält. Dabei ist die Pelotte auf der Spange oder der Bandage bevorzugt so positioniert, dass die Längsseiten der Pelotte nahezu parallel zu den Längsseiten der Spange oder der Bandage, die den Arm umfassen, verlaufen. Dabei kann in einer bevorzugten Ausführungsform vorgesehen sein, dass die Pelotte reversibel mit dem Pelottenträger verbunden ist, auch wenn eine grundlegende Umpositionierung der Pelotte durch Verwendung der erfindungsgemäßen Pelotte nicht mehr notwendig ist. Jedoch ist eine reversible Befestigung vorteilhaft, um eine minimale Nachjustierung der Pelottenposition auf dem Pelottenträger zu ermöglichen. Bevorzugt ist also die Pelotte, insbesondere die zweite Fläche der Pelotte, über ein Haltemittel, insbesondere lösbares Haltemittel mit dem Pelottenträger verbunden.

Eine zweckmäßige Befestigung der Pelotte kann durch einen die Haltemittel bildenden Klettverschluss bestehen, der zum einen an dem Pelottenträger und zum anderen an der Pelotte oder der Umhüllung der Pelotte angebracht ist, wobei die zweite Fläche der Pelotte mit dem Pelottenträger verbunden wird. Es ist aber auch möglich, die Haltemittel als Raststifte und diese aufnehmenden Bohrungen zu bilden. Dabei können die Raststifte entweder an der Pelotte und die zugehörigen Bohrungen an der Spange ausgebildet sein, es ist aber auch möglich, die Raststifte an der Spange und die wiederaufnehmenden Bohrungen an der Pelotte auszubilden. In einer bevorzugten Ausführungsform ist die Pelotte aber über eine Klettverbindung mit dem Pelottenträger reversibel verbunden.

In einer bevorzugten Ausführungsform ist die Pelotte in einer weichen, textilen Umhüllung eingefasst und die Umhüllung ist über eine Klettverbindung mit dem Pelottenträger reversibel verbunden.

Bevorzugt ist eine Epicondylitis-Spange mit einer ein Federband enthaltenden Klammer, die durch ein stufenlos verstellbares Halteband zusammenziehbar ist, wobei an dem einen Ende der Klammer an der Innenseite der Spange eine erfindungsgemäße Pelotte angeordnet ist, wobei die Pelotte bevorzugt reversibel mit der Innenseite der Spange verbunden ist.

Bevorzugt handelt es sich bei der Epicondylitis-Spange, der Epicondylitis-Bandage oder Epicondylitis-Orthese um ein Sportprodukt, das bevorzugt nicht medizinisch verwendet wird. Es ist jedoch auch eine medizinische Verwendung möglich.

Die vorliegende Erfindung betrifft auch die Verwendung einer erfindungsgemäßen Pelotte, einer erfindungsgemäßen Epicondylitis-Spange, eine erfindungsgemäße Epicondylitis-Bandage oder eine erfindungsgemäße Epicondylitis-Orthese als Sportprodukt oder beim Ausüben von Sport, beispielsweise beim Tennisspielen oder Golfspielen.

Die vorliegende Erfindung betrifft auch die Verwendung einer erfindungsgemäßen Pelotte, einer erfindungsgemäßen Epicondylitis-Spange, eine erfindungsgemäße Epicondylitis-Bandage oder eine erfindungsgemäße Epicondylitis-Orthese zur Prävention vor Beschwerden im Ellenbogenbereich, insbesondere zur Prävention vor einem Tennisellenbogen und/oder vor einem Golferellenbogen.

Die vorliegende Beschreibung offenbart auch die Verwendung einer erfindungsgemäßen Pelotte, einer erfindungsgemäßen Epicondylitis-Spange, eine erfindungsgemäße Epicondylitis-Bandage oder eine erfindungsgemäße Epicondylitis-Orthese zur Behandlung eines Tennisellenbogens und/oder eines Golferellenbogens. Die vorliegende Beschreibung offenbart auch ein Verfahren zur Behandlung eines Tennisellenbogens und/oder eines Golferellenbogens, bei dem eine erfindungsgemäße Pelotte, beispielsweise über eine erfindungsgemäße Epicondylitis-Spange, eine erfindungsgemäße Epicondylitis-Bandage oder eine erfindungsgemäße Epicondylitis-Orthese auf den zu behandelnden Ellenbogen angedrückt wird, sodass die fünf Druckpolster auf entsprechende Triggerpunkte drücken, insbesondere die außen am Ellenbogen befindlichen Triggerpunkte am Musculus brachioradialis und die innenliegenden Triggerpunkte des Musculus pronator teres, des Musculus palmaris longus, des Musculus flexor carpi ulnaris und/oder des Musculus flexor carpi radialis.

Weitere bevorzugte Ausführungsformen ergeben sich aus den Unteransprüchen.

Die vorliegende Erfindung wird anhand der im Folgenden beispielhaften Figuren erläutert, ohne dass der Gegenstand der Figuren einschränkend zu verstehen ist.

Es zeigen:
- Figur 1: die Aufsicht auf eine erfindungsgemäße Pelotte,
- Figur 2: die Schrägansicht einer erfindungsgemäßen Pelotte von einer Breitseite aus,
- Figur 3: die Schrägansicht einer erfindungsgemäßen Pelotte von einer Längsseite aus,
- Figur 4: eine erfindungsgemäße Pelotte auf einem Pelottenträger einer Epicondylitis-Spange oder Epicondylitis-Bandage,
- Figur 5: eine erfindungsgemäße Pelotte an einer bevorzugten Ausführungsform einer Epicondylitis-Spange.

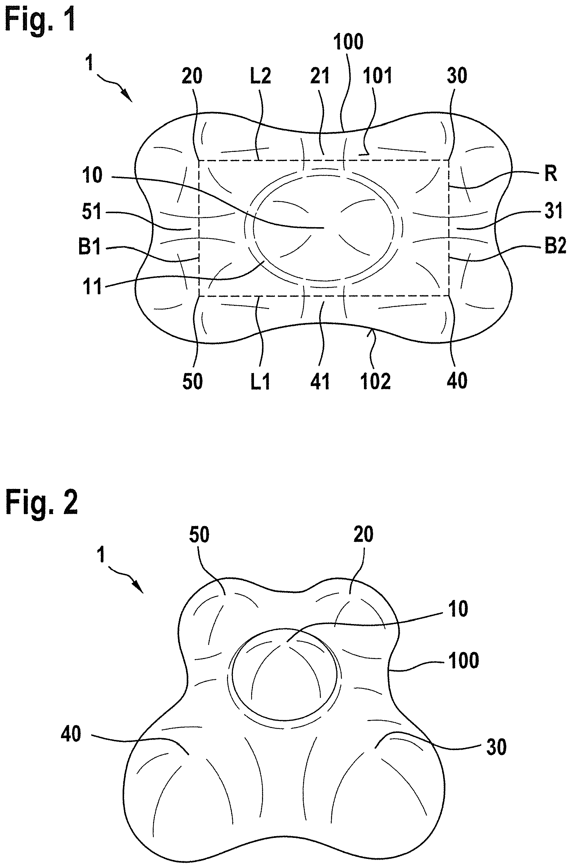

Figur 1 zeigt eine erfindungsgemäße Pelotte (1) in Aufsicht. Daher ist die erste Fläche (101) des Grundkörpers (100) der Pelotte (1) zu sehen, nicht jedoch die zweite Fläche (102). Aus dem Grundkörper (100) ragen fünf Druckpolster (10,20,30,40,50) heraus. Dabei bilden das zweite Druckpolster (20), das dritter Druckpolster (30), das vierte Druckpolster (40) und das fünfte Druckpolster (50) ein gedachtes Rechteck (R). In der Mitte des Rechtecks (R) liegt das erste Druckpolster (10). Das gedachte Rechteck (R), dessen Ecken durch die höchsten Punkte der vier außen liegenden Druckpolster (20,30,40,50) gebildet wird, hat zwei Längsseiten (L1,L2) mit einer Länge von etwa 4cm und zwei Breitseiten (B1,B2) mit einer Länge von etwa 2cm. Zwischen den Druckpolstern (10,20,30,40,50) befinden sich Vertiefungen (11,21,31,41,51).

Die gezeigte erfindungsgemäße Pelotte hat eine Form und eine Größe, bei der in vorteilhafter Weise die fünf Druckpolster (10,20,30,40,50) auf fünf Triggerpunkte im Ellenbogenbereich drücken können, und zwar sowohl auf Triggerpunkte an den äußeren Knochenvorsprüngen zur Behandlung des Tennisellenbogens als auch auf Triggerpunkte an den inneren Knochenvorsprüngen zur Behandlung des Golferellenbogens.

Somit ist die Behandlung eines Tennisellenbogens und/oder eines Golferellenbogens möglich.

Figur 2 zeigt die erfindungsgemäße Pelotte (1) mit dem Grundkörper (100) und den als Erhebungen ausgebildeten Druckpolstern (10,20,30,40,50) aus Figur 1 in Schrägansicht von der Breitseite aus.

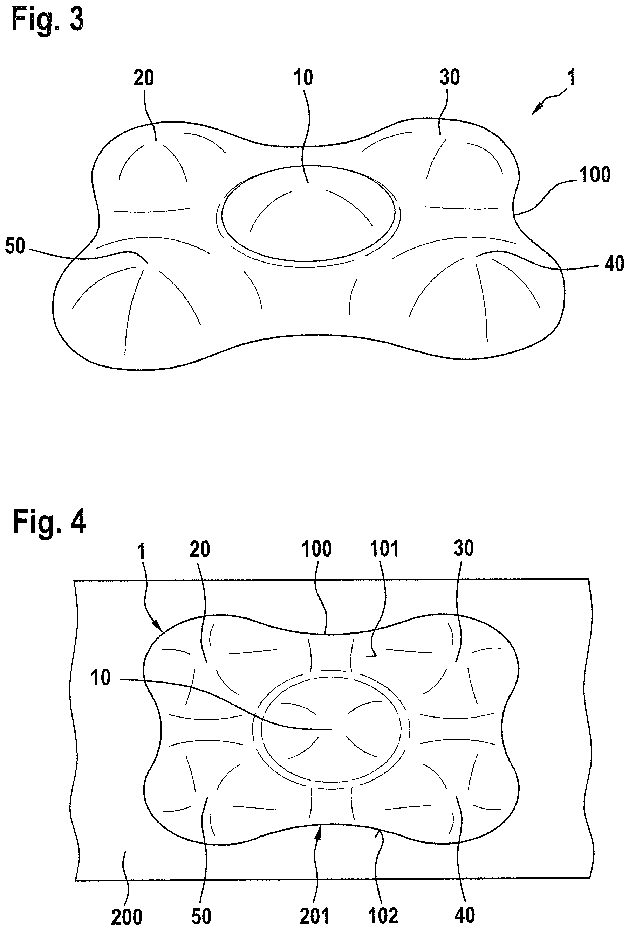

Figur 3 zeigt die erfindungsgemäße Pelotte (1) mit dem Grundkörper (100) und den als Erhebungen ausgebildeten Druckpolstern (10,20,30,40,50) aus Figur 1 in Schrägansicht von der Längsseite aus.

Figur 4 zeigt eine erfindungsgemäße Pelotte (1) mit dem Grundkörper (100) und den fünf Druckpolstern (10,20,30,40,50) der ersten Fläche (101) auf einem Pelottenträger (200). Die Pelotte (1) ist mit der nicht sichtbaren zweiten Fläche (102), die die Rückseite der Pelotte bildet, über eine nicht sichtbare Klettverbindung (201) aus einer Klettschicht und einer Flauschschicht mit dem Pelottenträger (200) reversibel verbunden. Der Pelottenträger (200) kann Bestandteil einer Epicondylitis-Spange, einer Epicondylitis-Bandage oder einer Epicondylitis-Orthese sein. Die Epicondylitis-Spange, Epicondylitis-Bandage oder Epicondylitis-Orthese wird, wie dem Fachmann bekannt ist, um den Unterarm im Bereich am Ellenbogen herumgelegt, sodass die Pelotte (1) mit den Druckpolstern (10,20,30,40,50) auf dortige Triggerpunkte drückt, wobei die Pelotte (1) durch den Pelottenträger (200) an den Arm angedrückt wird.

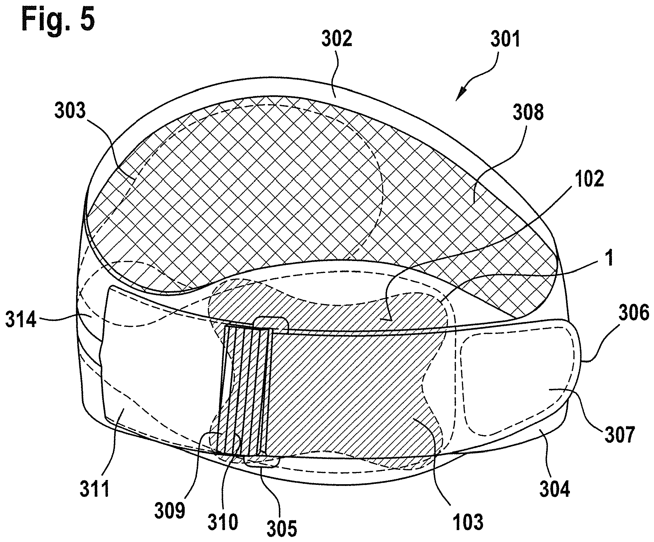

Figur 5 zeigt eine Epicondylitis-Spange (301) auf Basis der in der DE 197 16 705 C1 gezeigten Spangenausführung, die aus einer Klammer (302) mit einem darin enthaltenen Federband (303) besteht. Das Federband (303) ist in die Klammer (302) eingeklebt. Die Klammer (302) geht in das gegenüber der Klammer (302) schmalere Halteband (304) über, das in der geschlossenen gezeigten Darstellung durch das Umlenkelement (305) in Form einer gestreckten Öse geschlungen ist und mit seinem Ende (306) über den Anfang des Haltebands (304) gelegt ist, wo es mittels des Klettverschlusses (307) an dem Halteband (304) festgehalten wird. Die Klammer (302) ist auf ihrer Innenseite mit einer das Tragen der Epicondylitis-Spange erleichternden Textilauflage (308) (kreuzstraffiert) versehen. Das Umlenkelement (305) ist an der Klammer (302) über das Elastikband (309) befestigt, das mit mehreren Quernähten (310) versehen ist. Das Elastikband (309) verläuft auf seiner dem Umlenkelement abgewandten Seite in der auf die Klammer (302) aufgebrachten Tasche (311), in der es an der Klammer (302) befestigt ist. Die Klammer (302) umhüllt das in ihr enthaltende Federband (303) mit einem Überzug auf Textilmaterial, der in das Halteband (304) übergeht und für den Klettverschluss (307) mit seinen Klettelementen das zugehörige Flauschteil bildet. Das Federband (303) hat eine Schwächung (314), durch die das Federband (303) gegenüber seinen Endbereichen erheblich verschmälert ist. Aufgrund dieser Schwächung (314) des Federbands (303) gibt diese dem Federband (303) eine gewisse Scharnierwirkung im Bereich der Schwächung (314), sodass beim straffen Anziehen des Haltebands (304) der die Pelotte (1) tragende Teil der Klammer (302) sich gegenüber der sonstigen Rundung der Klammer (302) nach innen zu abbiegt und damit in der Lage ist, einen besonderen Druck auf den an dieser Stelle liegenden Teil des Unterarms auszuüben.

Gezeigt ist weiterhin eine gestrichelt gezeichnete erfindungsgemäße Pelotte (1), die in der geschlossen gezeichneten Lage der Epicondylitis-Spange (301) auf der Innenseite der Klammer (302) angeordnet und daher in Figur 5 nicht sichtbar dargestellt ist. Die Pelotte ist in einer textilen Umhüllung (103) eingefasst. Die textile Umhüllung ist über einen Klettverschluss (201) an der Klammer (302) befestigt, sodass die zweite Fläche (102) der Pelotte (1) an der Klammer (302) anliegt.

## Patentansprüche

1. Pelotte (1) mit einen Grundkörper, wobei der Grundkörper (100) eine erste Fläche (101) und eine der ersten Fläche gegenüberliegende zweite Fläche (102) aufweist, wobei die erste Fläche (101) des Grundkörpers (100) Druckpolster (10,20,30,40,50) aufweist, **dadurch gekennzeichnet, dass** die erste Fläche (101) des Grundkörpers (100) exakt fünf Druckpolster (10,20,30,40,50) aufweist, wobei das erste Druckpolster (10) im Mittelpunkt eines Rechtecks (R) liegt, das durch das zweite Druckpolster (20), das dritte Druckpolster (30), das vierte Druckpolster (40) und das fünfte Druckpolster (50) gebildet wird.

2. Pelotte nach Anspruch 1, wobei der Grundkörper (100) die Grundform des Rechtecks (R) aufweist, wobei das Rechtecks (R) abgerundete Ecken aufweist und wobei die vier abgerundeten Ecken des Grundkörpers durch das zweite Druckpolster (20), das dritte Druckpolster (30), das vierte Druckpolster (40) und das fünfte Druckpolster (50) gebildet werden.

3. Pelotte nach einem der vorhergehenden Ansprüche, wobei der Grundkörper (100) zwischen dem zweiten Druckpolster (20) und dem dritten Druckpolster (30), zwischen dem dritten Druckpolster (30) und dem vierten Druckpolster (40), zwischen dem vierten Druckpolster (40) und dem fünften Druckpolster (50) und zwischen dem fünften Druckpolster (50) und dem zweiten Druckpolster (20) jeweils eine Einbuchtung aufweist.

4. Pelotte nach einem der vorhergehenden Ansprüche, wobei der Grundkörper (100) zwischen den Druckpolstern (10,20,30,40,50) Vertiefungen (11,21,31,41,51) aufweist.

5. Pelotte nach einem der vorhergehenden Ansprüche, wobei das Rechteck (R) zwei Längsseiten (L1,L2) und zwei Breitseiten (B1,B2) aufweist und wobei die Längsseiten (L1,L2) mindestens 1,2 mal bis höchstens 2,5 mal so lang sind wie die Breitseiten (B1,B2).

6. Pelotte nach einem der vorhergehenden Ansprüche, wobei die fünf Druckpolster (10,20,30,40,50) im Längsschnitt eine runde oder ellipsoide Schnittfläche aufweisen und wobei sich die fünf Druckpolster (10,20,30,40,50) aus dem Grundkörper (100) blasenartig herausragen.

7. Pelotte nach einem der vorhergehenden Ansprüche, wobei die Pelotte aus einem viscoelastischen Material gefertigt ist.

8. Epicondylitis-Spange, Epicondylitis-Bandage oder Epicondylitis-Orthese umfassend eine Pelotte nach einem der vorhergehenden Ansprüche und einen Pelottenträger (200).

9. Epicondylitis-Spange, Epicondylitis-Bandage oder Epicondylitis-Orthese umfassend einen Pelottenträger (200). und eine Pelotte (1) mit einen Grundkörper, wobei der Grundkörper (100) eine erste Fläche (101) und eine der ersten Fläche gegenüberliegende zweite Fläche (102) aufweist, wobei die erste Fläche (101) des Grundkörpers (100) Druckpolster (10,20,30,40,50) aufweist, **dadurch gekennzeichnet, dass** die erste Fläche (101) des Grundkörpers (100) fünf Druckpolster (10,20,30,40,50) aufweist, wobei das erste Druckpolster (10) im Mittelpunkt eines Rechtecks (R) liegt, das durch das zweite Druckpolster (20), das dritte Druckpolster (30), das vierte Druckpolster (40) und das fünfte Druckpolster (50) gebildet wird, wobei der Grundkörper (100) die Grundform des Rechtecks (R) aufweist, wobei das Rechtecks (R) abgerundete Ecken aufweist und wobei die vier abgerundeten Ecken des Grundkörpers durch das zweite Druckpolster (20), das dritte Druckpolster (30), das vierte Druckpolster (40) und das fünfte Druckpolster (50) gebildet werden.

10. Epicondylitis-Spange, Epicondylitis-Bandage oder Epicondylitis-Orthese nach Anspruch 9, wobei der Grundkörper (100) zwischen dem zweiten Druckpolster (20) und dem dritten Druckpolster (30), zwischen dem dritten Druckpolster (30) und dem vierten Druckpolster (40), zwischen dem vierten Druckpolster (40) und dem fünften Druckpolster (50) und zwischen dem fünften Druckpolster (50) und dem zweiten Druckpolster (20) jeweils eine Einbuchtung aufweist.

11. Epicondylitis-Spange, Epicondylitis-Bandage oder Epicondylitis-Orthese nach Anspruch 9 oder Anspruch 10, wobei der Grundkörper (100) zwischen den Druckpolstern (10,20,30,40,50) Vertiefungen (11,21,31,41,51) aufweist.

12. Epicondylitis-Spange, Epicondylitis-Bandage oder Epicondylitis-Orthese nach einem der Ansprüche 9 bis 11, wobei das Rechteck (R) zwei Längsseiten (L1,L2) und zwei Breitseiten (B1,B2) aufweist und wobei die Längsseiten (L1,L2) mindestens 1,2 mal bis höchstens 2,5 mal so lang sind wie die Breitseiten (B1,B2).

13. Epicondylitis-Spange, Epicondylitis-Bandage oder Epicondylitis-Orthese nach einem der Ansprüche 9 bis 12, wobei die fünf Druckpolster (10,20,30,40,50) im Längsschnitt eine runde oder ellipsoide Schnittfläche aufweisen und wobei sich die fünf Druckpolster (10,20,30,40,50) aus dem Grundkörper (100) blasenartig herausragen und/oder wobei die Pelotte aus einem viscoelastischen Material gefertigt ist..

14. Epicondylitis-Spange, Epicondylitis-Bandage oder Epicondylitis-Orthese nach einem der Ansprüche 8 bis 13, wobei die Pelotte über eine Klettverbindung (201) mit dem Pelottenträger (200) reversibel verbunden ist.

15. Epicondylitis-Spange, Epicondylitis-Bandage oder Epicondylitis-Orthese nach einem der Ansprüche 8 bis 14, wobei die Pelotte in einer weichen Umhüllung (103) eingefasst ist und die Umhüllung (103) über eine Klettverbindung (201) mit dem Pelottenträger (200) reversibel verbunden ist.

## Claims

1. A pressure pad (1) having a base body, wherein the base body (100) has a first surface (101) and a second surface (102) opposite the first surface, wherein the first surface (101) of the base body (100) has pressure cushions (10,20,30,40,50), **characterized in in that** the first surface (101) of the base body (100) has exactly five pressure cushions (10, 20, 30, 40, 50), wherein the first pressure cushion (10) is located in the center of a rectangle (R) that is formed by the second pressure cushion (20), the third pressure cushion (30), the fourth pressure cushion (40) and the fifth pressure cushion (50).

2. The pressure pad according to claim 1, wherein the base body (100) has the basic shape of the rectangle (R), wherein the rectangle (R) has rounded corners and wherein the four rounded corners of the base body are formed by the second pressure cushion (20), the third pressure cushion (30), the fourth pressure cushion (40) and the fifth pressure cushion (50).

3. The pressure pad according to one of the preceding claims, wherein the base body (100) respectively has a recess between the second pressure cushion (20) and the third pressure cushion (30), between the third pressure cushion (30) and the fourth pressure cushion (40), between the fourth pressure cushion (40) and the fifth pressure cushion (50), and between the fifth pressure cushion (50) and the second pressure cushion (20).

4. The pressure pad according to one of the preceding claims, wherein the base body (100) has indentations (11,21,31,41,51) between the pressure cushions (10,20,30,40,50).

5. The pressure pad according to one of the preceding claims, wherein the rectangle (R) has two longitudinal sides (L1,L2) and two broad sides (B1,B2) and wherein the longitudinal sides (L1,L2) are at least 1.2 times to at most 2.5 times as long as the broad sides (B1,B2).

6. The pressure pad according to one of the preceding claims, wherein the five pressure cushions (10,20,30,40,50) have a round or ellipsoidal interface in longitudinal section and wherein the five pressure cushions (10,20,30,40,50) protrude from the base body (100) in a bubble-like manner.

7. The pressure pad according to one of the preceding claims, wherein the pressure pad is made of a viscoelastic material.

8. An epicondylitis brace, epicondylitis bandage or epicondylitis orthosis comprising a pressure pad according to one of the preceding claims and a pressure pad carrier (200).

9. An epicondylitis brace, epicondylitis bandage or epicondylitis orthosis comprising a pressure pad carrier (200) and a pressure pad (1) having a base body, wherein the base body (100) has a first surface (101) and a second surface (102) opposite the first surface, wherein the first surface (101) of the base body (100) has pressure cushions (10,20,30,40,50), **characterized in that** the first surface (101) of the base body (100) has five pressure cushions (10,20,30,40,50), wherein the first pressure cushion (10) is located in the center of a rectangle (R), that is formed by the second pressure cushion (20), the third pressure cushion (30), the fourth pressure cushion (40) and the fifth pressure cushion (50), wherein the base body (100) has the basic shape of the rectangle (R), wherein the rectangle (R) has rounded corners and wherein the four rounded corners of the base body are formed by the second pressure cushion (20), the third pressure cushion (30), the fourth pressure cushion (40) and the fifth pressure cushion (50).

10. The epicondylitis brace, epicondylitis bandage or epicondylitis orthosis according to claim 9, wherein the base body (100) respectively has a recess between the second pressure cushion (20) and the third pressure cushion (30), between the third pressure cushion (30) and the fourth pressure cushion (40), between the fourth pressure cushion (40) and the fifth pressure cushion (50) and between the fifth pressure cushion (50) and the second pressure cushion (20).

11. The epicondylitis brace, epicondylitis bandage or epicondylitis orthosis according to claim 9 or claim 10, wherein the base body (100) has indentations (11,21,31,41,51) between the pressure cushions (10,20,30,40,50).

12. The epicondylitis brace, epicondylitis bandage or epicondylitis orthosis according to one of claims 9 to 11, wherein the rectangle (R) has two longitudinal sides (L1,L2) and two broad sides (B1,B2) and wherein the longitudinal sides (L1,L2) are at least 1.2 times to at most 2.5 times as long as the broad sides (B 1,B2).

13. The epicondylitis brace, epicondylitis bandage or epicondylitis orthosis according to one of claims 9 to 12, wherein the five pressure cushions (10,20,30,40,50) have a round or ellipsoidal interface in longitudinal section and wherein the five pressure cushions (10,20,30,40,50) protrude from the base body (100) in a bubble-like manner and/or wherein the pressure pad is made of a viscoelastic material.

14. The epicondylitis brace, epicondylitis bandage or epicondylitis orthosis according to one of claims 8 to 13, wherein the pressure pad is reversibly attached to the pressure pad carrier (200) via a hook-and-loop connection (201).

15. The epicondylitis brace, epicondylitis bandage or epicondylitis orthosis according to one of claims 8 to 14, wherein the pressure pad is enclosed in a soft casing (103) and the casing (103) is reversibly attached to the pressure pad carrier (200) via a hook-and-loop connection (201).

## Revendications

1. Une pelotte (1) ayant un corps de base, dans laquelle le corps de base (100) a une première surface (101) et une deuxième surface (102) opposée à la première surface, dans laquelle la première surface (101) du corps de base (100) a des coussins de pression (10, 20, 30, 40, 50), **caractérisée en ce que** la première surface (101) du corps de base (100) a exactement cinq coussins de pression (10, 20, 30, 40, 50), dans lequel le premier coussin de pression (10) est situé au centre d'un rectangle (R) qui est formé par le deuxième coussin de pression (20), le troisième coussin de pression (30), le quatrième coussin de pression (40) et le cinquième coussin de pression (50).

2. La pelotte selon la revendication 1, dans lequel le corps de base (100) a la forme de base du rectangle (R), dans lequel le rectangle (R) a des coins arrondis et dans lequel les quatre coins arrondis du corps de base sont formés par le deuxième coussin de pression (20), le troisième coussin de pression (30), le quatrième coussin de pression (40) et le cinquième coussin de pression (50).

3. La pelotte selon l'une des revendications précédentes, dans lequel le corps de base (100) a respectivement un renfoncement entre le deuxième coussin de pression (20) et le troisième coussin de pression (30), entre le troisième coussin de pression (30) et le quatrième coussin de pression (40), entre le quatrième coussin de pression (40) et le cinquième coussin de pression (50), et entre le cinquième coussin de pression (20).

4. La pelotte selon l'une des revendications précédentes, dans lequel le corps de base (100) a des indentations (11,21,31,41,51) entre les coussins de pression (10,20,30,40,50).

5. La pelotte selon l'une des revendications précédentes, dans lequel le rectangle (R) a deux côtés longitudinaux (L1,L2) et deux côtés grands (B1,B2) et dans lequel les côtés longitudinaux (L1,L2) sont au moins 1,2 fois à au plus 2,5 fois plus longs que les côtés grands (B1,B2).

6. La pelotte selon l'une des revendications précédentes, dans lequel les cinq coussins de pression (10,20,30,40,50) ont une interface ronde ou ellipsoïdale en coupe longitudinale et dans lequel les cinq coussins de pression (10,20,30,40,50) dépassent du corps de base (100) à la manière d'une bulle.

7. La pelotte selon l'une des revendications précédentes, dans lequel la pelotte est constituée d'un matériau viscoélastique.

8. Un bracelet d'épicondylite, un bandage d'épicondylite ou une orthèse d'épicondylite comprenant une pelotte selon l'une des revendications précédentes et un support de pelotte (200).

9. Un bracelet d'épicondylite, un bandage d'épicondylite ou une orthèse d'épicondylite comprenant un support de pelotte (200) et une pelotte (1) ayant un corps de base, dans lequel le corps de base (100) a une première surface (101) et une deuxième surface (102) opposée à la première surface, dans lequel la première surface (101) du corps de base (100) a des coussins de pression (10,20,30,40,50), **caractérisé en ce que** la première surface (101) du corps de base (100) a cinq coussins de pression (10,20,30,40,50), dans lequel le premier coussin de pression (10) est situé au centre d'un rectangle (R), qui est formé par le deuxième coussin de pression (20), le troisième coussin de pression (30), le quatrième coussin de pression (40) et le cinquième coussin de pression (50), dans lequel le corps de base (100) a la forme de base du rectangle (R), dans lequel le rectangle (R) a des coins arrondis et dans lequel les quatre coins arrondis du corps de base sont formés par le deuxième coussin de pression (20), le troisième coussin de pression (30), le quatrième coussin de pression (40) et le cinquième coussin de pression (50).

10. Le bracelet d'épicondylite, le bandage d'épicondylite ou l'orthèse d'épicondylite selon la revendication 9, dans laquelle le corps de base (100) a respectivement un renfoncement entre le deuxième coussin de pression (20) et le troisième coussin de pression (30), entre le troisième coussin de pression (30) et le quatrième coussin de pression (40), entre le quatrième coussin de pression (40) et le cinquième coussin de pression (50) et entre le cinquième coussin de pression (50) et le deuxième coussin de pression (20).

11. Le bracelet d'épicondylite, le bandage d'épicondylite ou l'orthèse d'épicondylite selon la revendication 9 ou la revendication 10, dans laquelle le corps de base (100) a des indentations (11,21,31,41,51) entre les coussins de pression (10,20,30,40,50).

12. Le bracelet d'épicondylite, le bandage d'épicondylite ou l'orthèse d'épicondylite selon l'une des revendications 9 à 11, dans laquelle le rectangle (R) a deux côtés longitudinaux (L1,L2) et deux côtés grands (B1,B2) et dans laquelle les côtés longitudinaux (L1,L2) sont au moins 1,2 fois à au plus 2,5 fois plus longs que les côtés grands (B 1,B2).

13. Le bracelet d'épicondylite, le bandage d'épicondylite ou l'orthèse d'épicondylite selon l'une des revendications 9 à 12, dans laquelle les cinq coussins de pression (10,20,30,40,50) ont une interface ronde ou ellipsoïdale en coupe longitudinale et dans laquelle les cinq coussins de pression (10,20,30,40,50) dépassent du corps de base (100) à la manière d'une bulle et/ou dans laquelle la pelotte est constituée d'un matériau viscoélastique.

14. Le bracelet d'épicondylite, le bandage d'épicondylite ou l'orthèse d'épicondylite selon l'une des revendications 8 à 13, dans lequel la pelotte est fixée de manière réversible au support de pelotte (200) par une connexion auto-agrippante (201).

15. Le bracelet d'épicondylite, le bandage d'épicondylite ou l'orthèse d'épicondylite selon l'une des revendications 8 à 14, dans laquelle la pelotte est enfermé dans une enveloppe souple (103) et l'enveloppe (103) est fixée de manière réversible au support de pelotte (200) via une connexion auto-agrippante (201).
